# EUROPEAN PATENT APPLICATION

(11) **EP 4 843 541 A2**
(43) Date of publication of application: **25.11.2026**
(21) Application number: 26190251.4
(22) Date of filing: 08.02.2022
(51) Int. Cl.: A61F 2/856

(54) **A CONSTRAINT ARRANGEMENT FOR A STENT-GRAFT LOADED ONTO A DELIVERY SYSTEM**

(30) Priority: 02.03.2021 AU 2021201336
(62) Document number(s) of the earlier application(s) in accordance with Art. 76 EPC: 22155713.5 / 4 059 480
(71) Applicant: Cook Medical Technologies LLC, Bloomington, IN 47404 (US)
(72) Inventor: DUCKE, Werner, Rochedale South, 4123 (AU)
(74) Representative: Williams Powell

(57) **Abstract**

A constraint arrangement (1) for a stent-graft (10) loaded onto a delivery system (5) is disclosed. The constraint arrangement (1) comprises: a suture (100) extending circumferentially around an outside surface (22) of a graft from a first fixed end (103) fixed to a stent-graft (10) to a positionable region (60), and through the graft at a first constraining perforation (62) into the lumen (25) and returning out of the lumen (25) to the outside surface (22) of the graft at a second constraining perforation (64) to a second fixed end (108) fixed to the stent-graft (10), thereby forming a loop (120) of the suture (100) within a lumen (25) of the graft, the loop (120) wrapped circumferentially around a cannula (80) such that a pair of legs (122, 127) of the loop (120) hold the positionable region with respect to the cannula (80), wherein a bight (225) of the loop (120) extends through a gap between the legs (122, 127) and wherein the release wire (90) extends through the bight (225) thereby capturing the bight (225) and releaseably securing the loop (120) around the cannula (80).

## Description

### FIELD OF DISCLOSURE

This disclosure relates to medical devices and more particularly to stent-grafts mountable onto a deployment device for endovascular introduction.

### BACKGROUND OF THE DISCLOSURE

This disclosure will be particularly discussed in relation to stent-grafts for placement into the aorta for the treatment of aneurysms. The disclosure, however, is not so restricted and may be applied to stent-grafts for placement in any lumen of the human or animal body.

The accurate positioning of stent-grafts is very important for a number of reasons, including in many cases the need to avoid occlusion of branch arteries. Positioning is complicated however because the diameter of a stent-graft is typically deliberately made larger than the diameter into which it is to be placed to allow for accurate sealing against the vessel wall, possible errors in sizing and subsequent relaxation of the vessel wall.

Once released from a delivery device, a stent-graft with self-expanding stents will take up a position against the vessel wall and it will be difficult if not impossible to reposition it. This can be dangerous if, for instance, renal arteries are occluded.

Many cases of aneurism repair require the accurate placement of stent-graft features such as a fenestration or scallop.

### SUMMARY

Aspects of the present invention seek to provide an improved constraint arrangement for a stent-graft loaded onto a delivery system.

Embodiments seek to address difficulties described above while at the same time improving safety and simplifying assembly or to at least provide a useful alternative assembly technique.

Throughout this specification the term distal with respect to a portion of the aorta, a deployment device or a prosthesis is the end of the aorta, deployment device or prosthesis further away in the direction of blood flow away from the heart and the term proximal means the portion of the aorta, deployment device or end of the prosthesis nearer to the heart. When applied to other vessels, similar terms such as caudal and cranial should be understood.

According to a first aspect of the disclosure, there is provided a constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
a suture extending circumferentially around an outside surface of the graft from a first fixed end fixed to the stent-graft to the positionable region, and through the graft at a first constraining perforation into the lumen and returning out of the lumen to the outside surface of the graft at a second constraining perforation to a second fixed end fixed to the stent-graft, thereby forming a loop of the suture within the lumen,
the loop wrapped circumferentially around the cannula such that a pair of legs of the loop hold the positionable region with respect to the cannula,
wherein a bight of the loop extends through a gap between the legs and wherein the release wire extends through the bight thereby capturing the bight and releaseably securing the loop around the cannula.

In one form, the release wire is retractable from the bight of the loop thereby releasing the bight such that the positionable region is not held with respect to the cannula.

In one form, the constraint arrangement is arranged and constructed such that expansion of the stent-graft, when the release wire is in the retracted condition, increases the circumferential length between the fixed end of the suture and the constraining perforation thereby creating tension in the suture, the tension acting to retract the loop towards an inner surface of the graft.

In one form, the suture penetrates through the graft into and then out of the lumen at least once between the first fixed end and the first constraining perforation.

In one form, the release wire repeatedly loops over the cannula along a longitudinal length of the stent-graft thereby securing the stent-graft to the cannula at a plurality of space-apart locations.

In one form, the release wire repeatedly penetrates the graft from within the lumen to an outside of the lumen and then penetrates the graft from the outside of the lumen to within the lumen.

In one form, the release wire is disposed helically along the length of the stent-graft.

In one form, the suture extends circumferentially around an outside surface of the graft over at least 45 degrees of arc from the fixed end fixed to the stent-graft to the positionable region, when the release wire has been retracted and the positionable region is not held with respect to the cannula.

In one form, the suture extends circumferentially around an outside surface of the graft over at least 75 degrees of the arc from the fixed end fixed to the stent-graft to the positionable region, when the release wire has been retracted and the positionable region is not held with respect to the cannula.

In one form, the feature is a fenestration.

In another form, the feature is a scalloped region.

According to a second aspect of the disclosure, there is provided a constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
a first suture extending circumferentially around an outside surface of the graft from a first fixed end fixed to the stent-graft to the positionable region, and through the graft and into the lumen and returning out of the lumen to the outside surface of the graft to a second fixed end fixed to the stent-graft, thereby forming a first loop of the first suture within the lumen,
a second suture extending circumferentially around the outside surface of the graft from a third fixed end fixed to the stent-graft to the positionable region, and into the lumen and returning out of the lumen to the outside surface of the graft to a fourth fixed end fixed to the stent-graft, thereby forming a second loop of the second suture within the lumen,
the second loop wrapped circumferentially around the cannula such that a second pair of legs of the second loop and a first pair of legs of the first loop hold the positionable region with respect to the cannula,
wherein a bight of the second loop extends through a gap between the first pair of legs of the first loop, and
wherein the release wire extends through the bight thereby capturing the bight and releaseably securing the first loop and the second loop around the cannula.

In one form, the release wire is retractable from the bight thereby releasing the bight such that the positionable region is not held with respect to the cannula.

In one form, the constraint arrangement is arranged and constructed such that expansion of the stent-graft, when the release wire is in the retracted condition, increases the circumferential length between the fixed end of the first suture and the positionable region thereby creating tension in the first suture, the tension acting to retract the first loop towards an inner surface of the graft.

In one form, the feature is a fenestration.

In another form, the feature is a scalloped region.

A detailed description of one or more embodiments of the disclosure is provided below along with accompanying figures that illustrate by way of example the principles of embodiments of the invention. While embodiments of the invention are described, it should be understood that the invention is not limited to any embodiment. On the contrary, the scope of the invention is limited only by the appended claims and the invention encompasses numerous alternatives, modifications and equivalents. For the purpose of example, numerous specific details are set forth in the following description in order to provide a thorough understanding of embodiments of the present invention.

The present invention may be practiced according to the claims without some or all of these specific details. For the purpose of clarity, technical material that is known in the technical fields related to the invention has not been described in detail so that the present invention is not unnecessarily obscured.

### BRIEF DESCRIPTION OF THE DRAWINGS

To assist with understanding, reference will now be made to the accompanying drawings which show preferred embodiments of the invention.
Figure 1A is a front view of a constraint arrangement for a stent-graft loaded onto a delivery system;
Figure 1B is a front view of another constraint arrangement for a stent-graft loaded onto a delivery system;
Figure 2A is a close-up view of Figure 1A from within a lumen of the stent-graft, showing routing of a suture being tied around a cannula;
Figure 2B is a similar view to that of Figure 2A, but with the suture tightened;
Figure 3 is a further view of the stent-graft shown in Figure 1A, again from inside the lumen, prior to securement;
Figure 4 is a close-up view of a portion of the constraint arrangement of Figures 1A, 2A, 2B and 3;
Figures 5A and 5B are diagrammatic views showing routing of the suture shown in Figure 1A in a constraining and non-constraining condition respectively;
Figures 6A, 6B and 6C are progressive illustrations showing the constraint arrangement of the previous figures being deployed and then released within a lumen of a patient;
Figure 7 is a front view of another constraint arrangement for a stent-graft loaded onto a delivery system, according to a second aspect of the disclosure;
Figure 8 is a further view of the stent-graft shown in Figure 7, from inside the lumen, prior to securement;
Figures 9A and 9B are diagrammatic views showing routing of the suture shown in Figure 7 in a constraining and non-constraining condition respectively; and
Figure 10 is a front view of a constraint arrangement for an alternative stent-graft loaded onto a delivery system.

### DESCRIPTION OF PREFERRED EMBODIMENTS

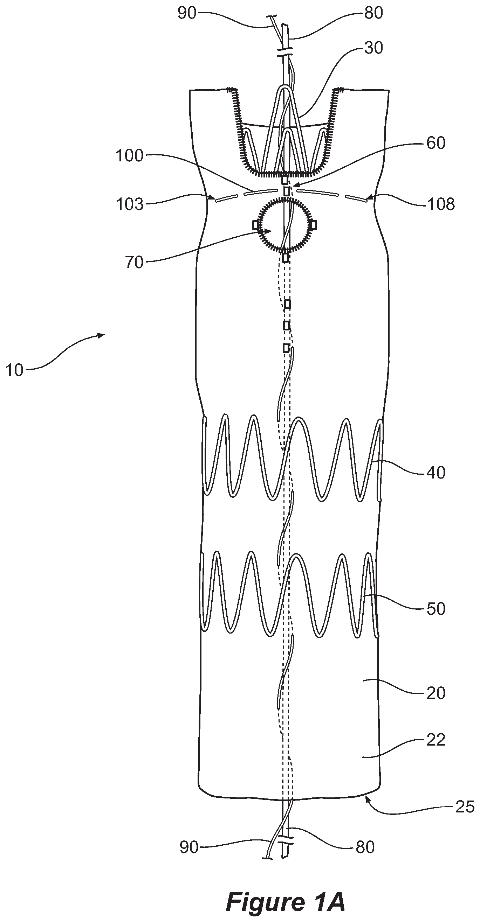

Referring to Figure 1A, a constraint arrangement for a stent-graft 10 loaded onto a delivery system according to a first embodiment of the disclosure is shown. The stent-graft has a graft 20 defining an elongate lumen 25, a plurality of stents 30, 40, 50 and at least one positionable region 60 adjacent to a feature, such as a fenestration 70. The delivery system has a cannula 80 and a release wire 90.

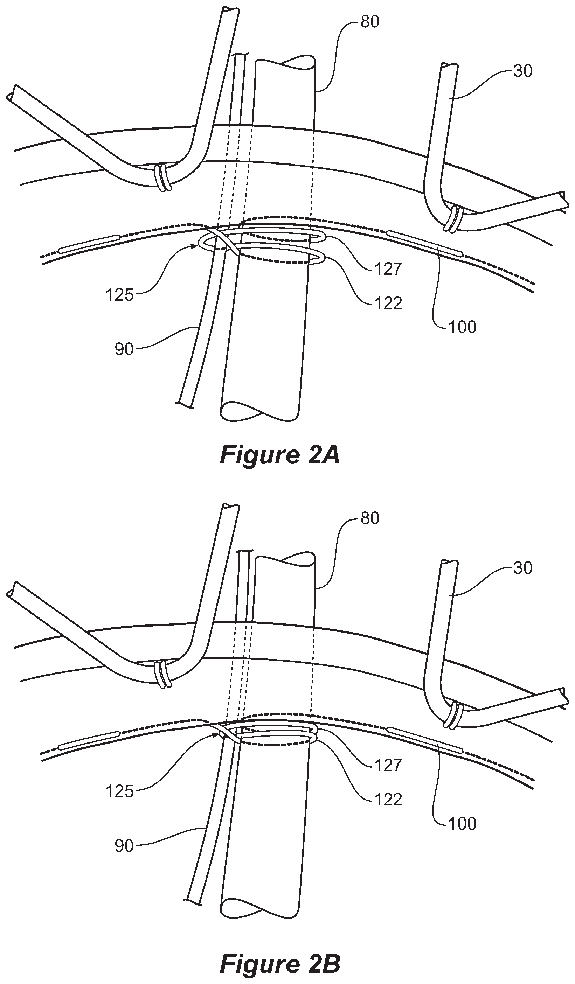

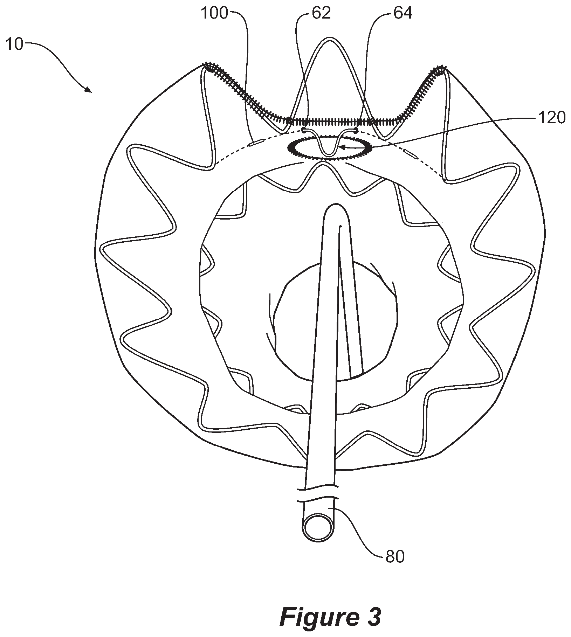

Now referring to Figures 1A, 2A, 2B and 3, it can be seen that the constraint arrangement comprises: a suture 100 extending circumferentially around an outside surface 22 of the graft from a first fixed end 103 fixed to the stent-graft to the positionable region 60, and through the graft 20 at a first constraining perforation 62 into the lumen and returning out of the lumen to the outside surface of the graft at a second constraining perforation 64 to a second fixed end 108 fixed to the stent-graft 10. A loop 120 of the suture is formed within the lumen. This loop 120 is shown in Figures 2A, 2B and 3.

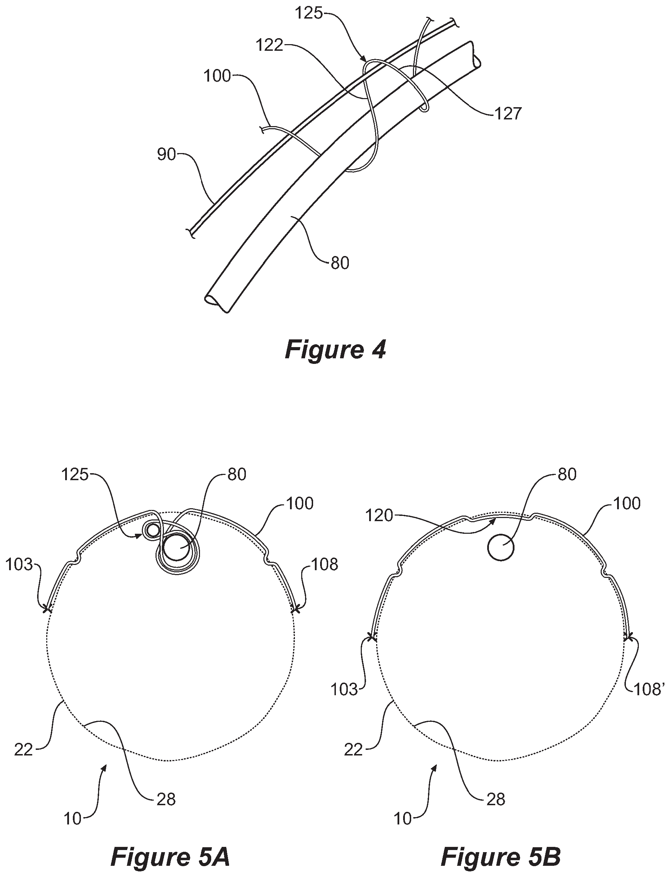

Referring to figures 4 and 5A, it can be seen that the loop 120 is wrapped circumferentially around the cannula 80 such that a pair of legs 122, 127 of the loop (most clearly shown in Figure 4) hold the positionable region 60 with respect to the cannula 80. A bight 125 of the loop 120 extends through a gap between the legs 122, 127 and the release wire 90 extends through the bight 125 thereby capturing the bight 125. Thus the release wire 90 releaseably secures the loop 120 around the cannula 80.

Referring to Figure 4, read with Figure 5A, the routing of the suture 100 with respect to the cannula 80 and the release wire 90 is clearly shown before tightening. As can be seen in Figure 5A, the graft 20 is securely held on the cannula 80 at a specific location or positionable region 60. Typically this positionable region 60 will be at, or adjacent to, an important feature of the stent-graft 10, such as a fenestration 70 or a scallop. The positionable region 60 is named a 'positionable' region, because it positionable by the clinician through manipulation of a handle connected to the cannula 80.

The release wire 90 is retractable from the bight 125 of the loop 120 thereby releasing the bight 125 such that the positionable region 60 is no longer held with respect to the cannula 80, as is shown in Figure 5B.

Using the constraint arrangement for a stent-graft described above, a certain portion of a stent-graft 10 can be controlled more accurately for positioning and alignment purposes. This assists clinicians in various ways. For instance it assists clinician conducting endovascular procedures for aneurism repair which require the accurate placement of stent-graft features such as a fenestration or scallop.

The ability to releasably constrain a required stent-graft feature, such as a fenestration, by utilizing existing components, such as a release or trigger wire is useful and helps keep the overall apparatus that is introduced into the vascular system of patient compact.

Considering Figure 5A and 5B together, it can be seen that the constraint arrangement is arranged and constructed such that expansion of the stent-graft 10, when the release wire is in the retracted condition, increases the circumferential length between the fixed end 103 of the suture 100 and the constraining perforation 62 (as shown on Figure 3) thereby creating tension in the suture 100. This tension acts to retract the loop 120 towards **an inner surface** 28 of the graft. This reduces the length of suture within the lumen and also reduces the likelihood of interference with normal blood slow once the procedure of delivering the stent-graft into a patient's anatomy is complete.

The suture 100 penetrates though the graft 20 into and then out of the lumen 25 at least once between the first fixed end 103 and the first constraining perforation 62 as can be seen reading Figures 1A and 3 together. With the embodiment shown in Figure 1A, most of the suture remains on the outside surface 22 of the stent-graft 10, thereby advantageously minimising the length of suture within the lumen 25.

Again referring to Figure 1A, it can be seen that the release wire 90 repeatedly loops over the cannula 80 along a longitudinal length of the stent-graft 10 thereby securing the stent-graft 10 to the cannula 80 at a plurality of spaced-apart locations. With the embodiment illustrated, the release wire repeatedly penetrates the graft from within the lumen to the outside of the lumen and then penetrates the graft from the outside of the lumen to within the lumen. The release wire is disposed helically along the length of the stent-graft, as can be seen in Figure 1A.

The release wire 90 retains the stent-graft to the cannula, or to a guide wire catheter, and includes a wire wound around the cannula and through the material of the stent-graft in a helical or spiral manner as described above and as illustrated in Figure 1A. The pitch of the helically wound release wire 90 can be between 20 to 40 mm for example.

The release wire 90 extends to a handle for instance, the handle manipulable by a clinician.

The suture 100 extends circumferentially around an outside surface 22 of the graft over at least 45 degrees of arc from the fixed end 103 fixed to the stent-graft 10 to the positionable region 60 when the release wire has been retracted and the positionable region is not held with respect to the cannula, such as is shown in Figure 5B. In the embodiment illustrated in Figures 1A, 5A and 5B, the suture extends circumferentially around an outside surface 22 of the graft over at least 75 degrees of arc from the fixed end 103 fixed to the stent-graft to the positionable region, when the release wire has been retracted and the positionable region is not held with respect to the cannula.

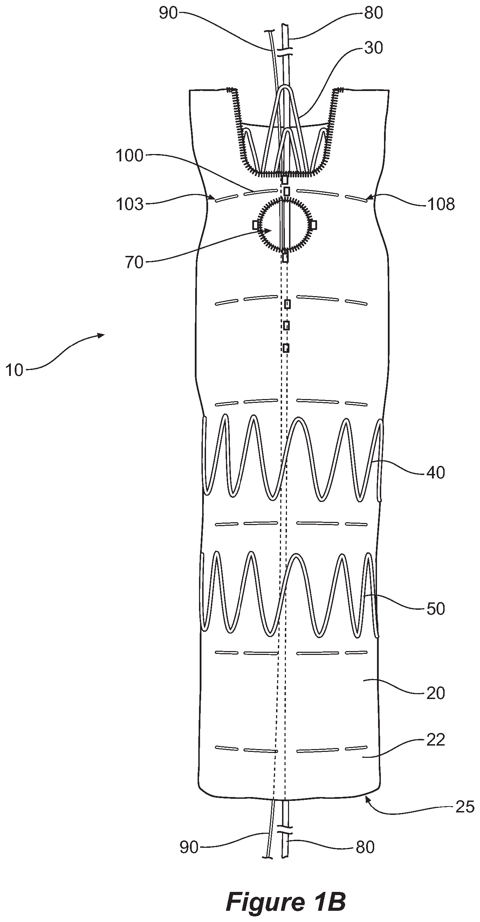

In another embodiment of the disclosure, shown in Figure 1B, a helically wound release wire is not employed. Instead, the release wire is relatively straight and attachment of the stent-graft 10 to the cannula along the length of the stent-graft 10 is achieved by using a plurality of sutures, each suture sewn like the suture 100 as described above.

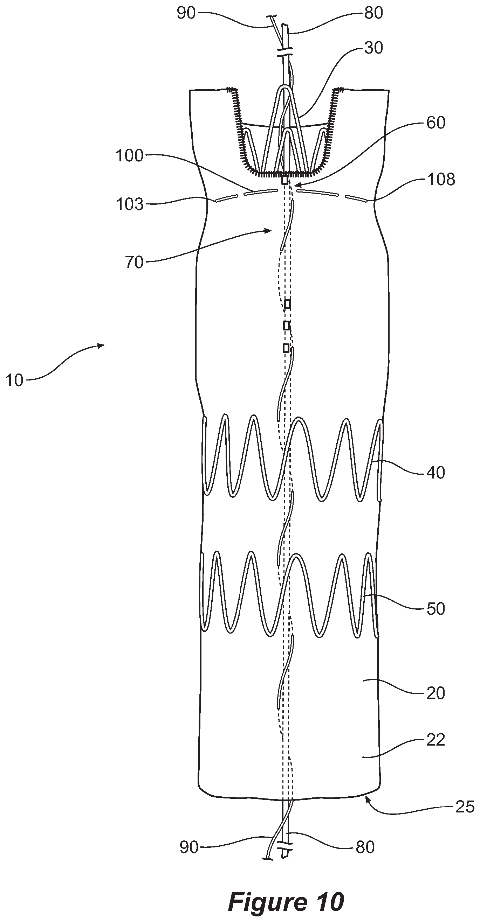

Referring now to Figure 10, a positionable region is adjacent to a scallop, allowing the clinician to precisely control the position of the scallop. In other respects the constraint arrangement is generally the same as that shown in Figures 1A, 1B, 2A, 2B, 3, 4, 5A and 5B, where the positionable region is adjacent to a fenestration.

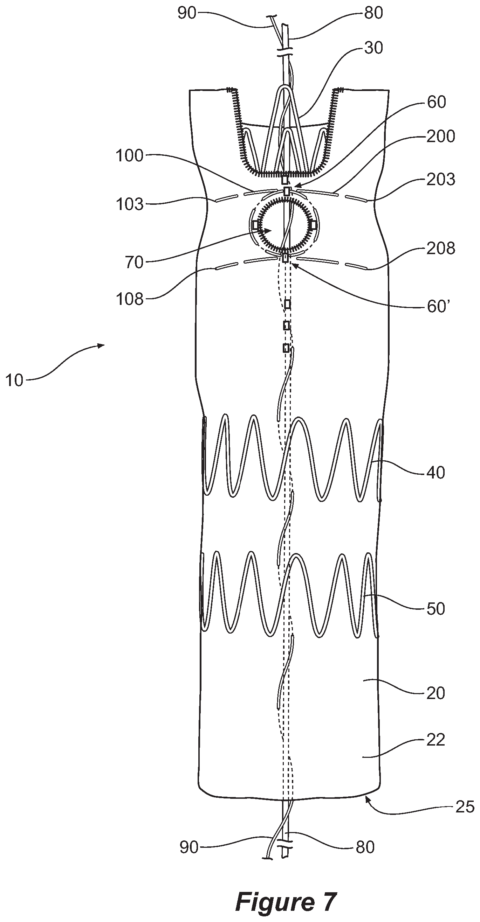

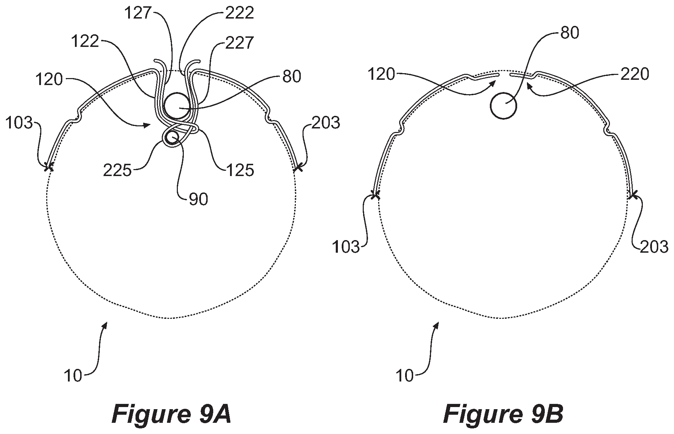

A second embodiment of the disclosure is shown in Figures 7 to 9B. In Figure 7, a constraint arrangement for a stent-graft 10 loaded onto a delivery system is shown. The stent-graft has a graft 20 defining an elongate lumen 25, a plurality of stents 30, 40, 50 and at least one positionable region 60 adjacent to a feature 70. The delivery system has a cannula 80 and a release wire 90. The constraint is similar to that of the constraint arrangement of the first embodiment of the disclosure in many ways, but differs in that two loops of suture are employed, rather than one, for each securement. Specifically, the constraint arrangement comprises a first suture 100 extending circumferentially around an outside surface 22 of the graft from a first fixed end 103 fixed to the stent-graft to the positionable region, and through the graft and into the lumen and returning out of the lumen to the outside surface of the graft to a second fixed end 108 fixed to the stent-graft, thereby forming a first loop 120 of the first suture within the lumen. This can be seen in Figures 9A and 9B were the first loop 120 is wrapped circumferentially (part circumferentially) around the cannula 80 such that a first pair of legs 122, 127 of the first loop 120 hold the positionable region 60 with respect to the cannula 80.

A second suture 200 also extends circumferentially (part circumferentially) around the outside surface 22 of the graft from a third fixed end 203 fixed to the stent-graft to the positionable region 60, and into the lumen 25 and returning out of the lumen 25 to the outside surface 22 of the graft to a fourth fixed end 208 fixed to the stent-graft, thereby forming a second loop 220 of the second suture within the lumen 25.

A second loop formed by the second suture is wrapped circumferentially around the cannula, such that a second pair of legs 222, 227 of the second loop 220 and a first pair of legs 122, 127 of the first loop 120 hold the positionable region 60 with respect to the cannula. A bight 225 of the second loop 220 extends through a gap between the first pair of legs 122, 127 of the second loop 220. The release wire 90 extends through the bight 225 thereby capturing the bight 225 and releaseably secures the first loop 120 and the second loop 220 around the cannula.

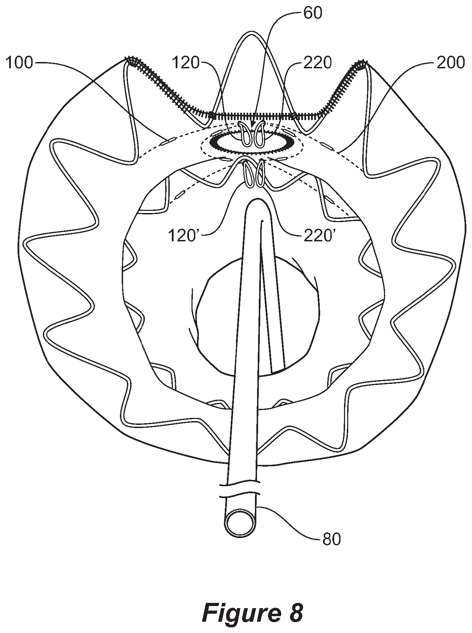

Referring to Figures 7 and 8, it can be seen that with this second embodiment there is also a second positionable region 60' located on the other side of a feature in the form of a fenestration 70. This second positionable region 60' is held to the cannula 80, providing further control to the clinician. It should be noted however that the double loop arrangement may be employed at just one positionable region 60 if desired.

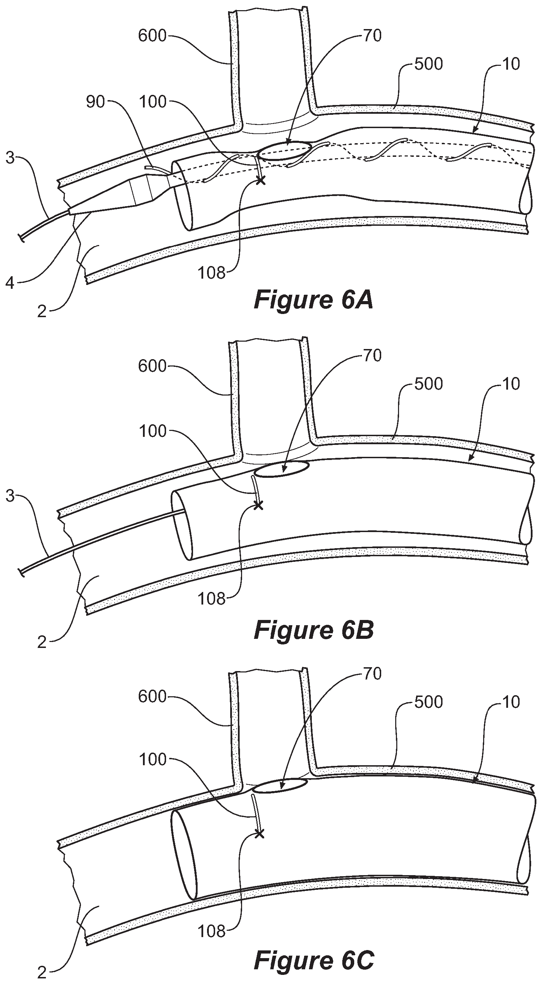

With all of the embodiments described above, the suture loops are greatly reduced (retracted) when the stent-graft is fully released and ballooned, as is shown progressively in Figures 6A to 6C. For example, this is achieved in the embodiment illustrated in Figures 1A to 5B by firstly weaving the loop material circumferentially around the stent-graft material for about 90 degrees either side of the feature, keeping it firm, but without reducing the stent-graft diameter, or at least not reducing it substantially, and then pulling the loop from the area of the feature that requires constraining. This way, the loop resumes its retracted condition along the circumference of the stent-graft after the stent-graft is released.

Now referring to the embodiment illustrated in Figure 1A again, a deployment sequence for the constraint arrangement will be described with reference to Figures 6A to 6C.

Figure 6A shows the constraint arrangement within a lumen 2 of a patient in a position where the feature, in this case a fenestration 70, is aligned with a target branch lumen 600 that branches from a main lumen 500. Typically the constraint arrangement and the stent-graft 10 will have reached this position using the Seldinger technique. This technique involves creating a surgical opening in the vascular system linked to the vessel of interest with a needle and inserting a wire guide into the vessel through a bore of the needle. For example, the femoral artery may be used to access the aorta. The needle can be withdrawn, leaving the wire guide in place. A delivery device is then inserted over the wire guide and into the vessel. The delivery device may be used in conventional fashion to insert into the blood vessel a stent-graft or other prostheses or devices. In Figure 6A, a guide wire 3 is shown over which a nose cone dilator 4 slides. Adjacent to the nose cone dilator 4 is the stent-graft 10 held to the cannula 80 by a helically wound release wire 90. The constraint arrangement described above with reference to Figures 1A, 2A, 2B, 3, 4, 5A and 5B is also partly visible in this Figure, with suture 100 and its first fixed end 103 clearly visible.

While in the position within the main lumen 500, which may be the aorta for instance, the clinician can manipulate the position of the fenestration 70 to align precisely with the opening of the branch lumen 600 (for example a renal artery). The clinician may then cannulate the target vessel with a wire and catheter prior to the final positioning and release of the stent-graft. Once this is complete, the release wire 90 can be retracted, releasing the positionable region 60 adjacent to the fenestration 70 and progressively releasing the stent-graft 10 from the cannula 80 until the position shown in Figure 6B is attained.

In a final step, the stent-graft 10 is typically further expanded by the deployment of a balloon from within the lumen 25 of the stent-graft 10 until the stent-graft 10 reaches the position shown in Figure 6C.

During the above step, the suture loop 120, or each suture loop is further retracted as the circumference of the stent-graft 10 increases.

Throughout this specification various indications have been given as to the scope of aspects of this invention but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

All optional and preferred features and modifications of the described embodiments and dependent claims and clauses below are usable in all aspects of the invention taught herein. Furthermore, the individual features of the dependent claims and clauses below, as well as all optional and preferred features and modifications of the described embodiments are combinable and interchangeable with one another.

The disclosures in Australian patent application number 2021201336, from which this application claims priority, in European patent application number 22155713.5, from which this application is divided, and in the abstract accompanying this application are incorporated herein by reference.
The skilled person will appreciate that the disclosure herein includes subject-matter relating to aspects of the invention in which there is provided a constraint arrangement as in any one of the following clauses.
1. A constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
   a suture extending circumferentially around an outside surface of the graft from a first fixed end fixed to the stent-graft to the positionable region, and through the graft at a first constraining perforation into the lumen and returning out of the lumen to the outside surface of the graft at a second constraining perforation to a second fixed end fixed to the stent-graft, thereby forming a loop of the suture within the lumen,
   the loop wrapped circumferentially around the cannula such that a pair of legs of the loop hold the positionable region with respect to the cannula,
   wherein a bight of the loop extends through a gap between the legs and wherein the release wire extends through the bight thereby capturing the bight and releaseably securing the loop around the cannula.
2. The constraint arrangement as recited in clause 1 wherein the release wire is retractable from the bight of the loop thereby releasing the bight such that the positionable region is not held with respect to the cannula.
3. The constraint arrangement as recited in any preceding clause arranged and constructed such that expansion of the stent-graft, when the release wire is in the retracted condition, increases the circumferential length between the fixed end of the suture and the constraining perforation thereby creating tension in the suture, the tension acting to retract the loop towards an inner surface of the graft.
4. The constraint arrangement as recited in any preceding clause wherein the suture penetrates through the graft into and then out of the lumen at least once between the first fixed end and the first constraining perforation.
5. The constraint arrangement as recited in any preceding clause wherein the release wire repeatedly loops over the cannula along a longitudinal length of the stent-graft thereby securing the stent-graft to the cannula at a plurality of space-apart locations.
6. The constraint arrangement as recited in any preceding clause wherein the release wire repeatedly penetrates the graft from within the lumen to an outside of the lumen and then penetrates the graft from the outside of the lumen to within the lumen.
7. The constraint arrangement as recited in any preceding clause wherein the release wire is disposed helically along the length of the stent-graft.
8. The constraint arrangement as recited in any preceding clause wherein the suture extends circumferentially around an outside surface of the graft over at least 45 degrees of arc from the fixed end fixed to the stent-graft to the positionable region, when the release wire has been retracted and the positionable region is not held with respect to the cannula.
9. The constraint arrangement as recited in any preceding clause wherein the suture extends circumferentially around an outside surface of the graft over at least 75 degrees of the arc from the fixed end fixed to the stent-graft to the positionable region, when the release wire has been retracted and the positionable region is not held with respect to the cannula.
10.The constraint arrangement as recited in any preceding clause wherein the feature is a fenestration.
11.The constraint arrangement as recited in any preceding clause wherein the feature is a scalloped region.
12.A constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
   a first suture extending circumferentially around an outside surface of the graft from a first fixed end fixed to the stent-graft to the positionable region, and through the graft and into the lumen and returning out of the lumen to the outside surface of the graft to a second fixed end fixed to the stent-graft, thereby forming a first loop of the first suture within the lumen,
   a second suture extending circumferentially around the outside surface of the graft from a third fixed end fixed to the stent-graft to the positionable region, and into the lumen and returning out of the lumen to the outside surface of the graft to a fourth fixed end fixed to the stent-graft, thereby forming a second loop of the second suture within the lumen,
   the second loop wrapped circumferentially around the cannula such that a second pair of legs of the second loop and a first pair of legs of the first loop hold the positionable region with respect to the cannula,
   wherein a bight of the second loop extends through a gap between the first pair of legs of the first loop, and
   wherein the release wire extends through the bight thereby capturing the bight and releaseably securing the first loop and the second loop around the cannula.
13.The constraint arrangement as recited in clause 12 wherein the release wire is retractable from the bight thereby releasing the bight such that the positionable region is not held with respect to the cannula.
14.The constraint arrangement as recited in clause 12 or 13 arranged and constructed such that expansion of the stent-graft, when the release wire is in the retracted condition, increases the circumferential length between the fixed end of the first suture and the positionable region thereby creating tension in the first suture, the tension acting to retract the first loop towards an inner surface of the graft.
15.The constraint arrangement as recited in clause 12, 13, or 14 wherein the feature is a fenestration or a scalloped region.

## Claims

1. A constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
a suture extending circumferentially around an outside surface of the graft from a first end to the positionable region, and through the graft into the lumen and returning out of the lumen to the outside surface of the graft to a second end, thereby forming a loop of the suture within the lumen,
the loop wrapped circumferentially around the cannula such that a pair of legs of the loop hold the positionable region with respect to the cannula,
wherein the release wire extends through a bight of the loop thereby capturing the bight and releaseably securing the loop around the cannula.

2. The constraint arrangement as claimed in claim 1 wherein the release wire is retractable from the bight of the loop thereby releasing the bight such that the positionable region is not held with respect to the cannula.

3. The constraint arrangement as claimed in any preceding claim, wherein the bight of the loop extends through a gap between the legs.

4. The constraint arrangement as claimed in any preceding claim, wherein the first and second ends are fixed to the stent graft.

5. The constraint arrangement as claimed in any preceding claim wherein the release wire repeatedly loops over the cannula along a longitudinal length of the stent-graft thereby securing the stent-graft to the cannula at a plurality of space-apart locations.

6. The constraint arrangement as claimed in any preceding claim wherein the release wire repeatedly penetrates the graft from within the lumen to an outside of the lumen and then penetrates the graft from the outside of the lumen to within the lumen.

7. The constraint arrangement as claimed in any preceding claim wherein the release wire is disposed helically along the length of the stent-graft.

8. The constraint arrangement as claimed in any preceding claim wherein the suture extends circumferentially around an outside surface of the graft over at least 45 degrees of arc from the first end to the positionable region, when the release wire has been retracted and the positionable region is not held with respect to the cannula.

9. The constraint arrangement as claimed in any preceding claim wherein the suture extends circumferentially around an outside surface of the graft over at least 75 degrees of the arc from the first end to the positionable region, when the release wire has been retracted and the positionable region is not held with respect to the cannula.

10. The constraint arrangement as claimed in any preceding claim wherein the feature is a fenestration or a scalloped region.

11. A constraint arrangement for a stent-graft loaded onto a delivery system, the stent-graft having a graft defining an elongate lumen, a plurality of stents and at least one positionable region adjacent to a feature, the delivery system having a cannula and a release wire, the constraint arrangement comprising:
a first suture extending circumferentially around an outside surface of the graft from a end to the positionable region, and through the graft and into the lumen and returning out of the lumen to the outside surface of the graft to a second end, thereby forming a first loop of the first suture within the lumen,
a second suture extending circumferentially around the outside surface of the graft from a third end to the positionable region, and into the lumen and returning out of the lumen to the outside surface of the graft to a fourth end, thereby forming a second loop of the second suture within the lumen,
the second loop wrapped circumferentially around the cannula such that a second pair of legs of the second loop and a first pair of legs of the first loop hold the positionable region with respect to the cannula,
wherein a bight of the second loop extends through a gap between the first pair of legs of the first loop, and
wherein the release wire extends through the bight thereby capturing the bight and releaseably securing the first loop and the second loop around the cannula.

12. The constraint arrangement as claimed in any claim 11, wherein the first, second, third, and fourth ends are fixed to the stent graft.

13. The constraint arrangement as claimed in claim 11 or 12 wherein the release wire is retractable from the bight thereby releasing the bight such that the positionable region is not held with respect to the cannula.

14. The constraint arrangement as claimed in claim 11, 12 or 13 arranged and constructed such that expansion of the stent-graft, when the release wire is in the retracted condition, increases the circumferential length between the fixed end of the first suture and the positionable region thereby creating tension in the first suture, the tension acting to retract the first loop towards an inner surface of the graft.

15. The constraint arrangement as claimed in claim 11, 12, 13, or 14 wherein the feature is a fenestration or a scalloped region.
